# FASCICULE DE BREVET EUROPEEN

(11) **EP 0 928 438 B1**
(45) Date de publication et mention de la délivrance du brevet: **04.09.2002**
(21) Numéro de dépôt: 97942071.8
(22) Date de dépôt: 22.09.1997
(51) Int. Cl.: G03B 42/02, G01T 1/29, A61B 6/00

(54) **DISPOSITIF AMOVIBLE DE PRISE D'IMAGES NUMERIQUES DESTINE A LA RADIOLOGIE**
ABNEHMBARE VORRICHTUNG ZUR DIGITALEN BILDERFASSUNG IN DER RADIOLOGIE
DETACHABLE DEVICE FOR RADIOLOGICAL DIGITAL IMAGING

(30) Priorité: 24.09.1996 FR 9611610
(43) Date de publication de la demande: 14.07.1999
(73) Titulaire: GE MEDICAL SYSTEMS SA, 78533 Buc Cedex (FR)
(72) Inventeur: HEIDSIECK, Robert, F-78150 Rocquencourt (FR); RIT, Vincent, F-59500 Douai (FR); PICARD, Catherine, F-92100 Boulogne (FR); BAUDET, Jean-Louis, F-75003 Paris (FR)
(74) Mandataire: Goode, Ian Roy
(86) Numéro de dépôt international: FR9701665
(87) Numéro de publication internationale: WO98013727

(56) Documents cités:
- EP-A- 0 714 038
- DE-A- 4 403 930

## Description

La présente invention a pour objet un dispositif de cassette amovible de prise d'images numériques destiné à la radiographie, par exemple à la mammographie par rayons X. Un tel dispositif de prise d'images est destiné à être inséré dans un appareil de radiologie comprenant une source de rayons X, un moyen de maintien de l'organe radiographié et un dispositif amovible de prise d'images.

On connaît, par exemple, des appareils de mammographie qui comprennent une source de rayons X disposée d'un côté de l'organe à radiographier, une table de support transparente aux rayons X, disposée de l'autre côté de l'organe à radiographier, un plateau de maintien réglable venant appliquer l'organe sur la table de support et un logement pour recevoir une cassette de prise d'images contenant un film impressionnable. Le logement est disposé dans la table de support.

Après qu'une image de l'organe a été prise, la cassette contenant le film impressionné est extraite de son logement et le film est développé.

De tels appareils servent en général à la recherche d'éventuels symptômes de cancer du sein. Une première opération consiste à effectuer un dépistage systématique qui ne nécessite que la prise d'un ou deux clichés. Si ces clichés révèlent des symptômes de cancer, on procède alors à un diagnostic plus approfondi qui nécessite un plus grand nombre de clichés, par exemple d'une zone particulière de l'organe, en vue de jouer sur le mode de représentation et de visualisation des images. Si le diagnostic révèle la présence d'un cancer, il peut être nécessaire d'effectuer une biopsie. On dispose alors un système de ponction sur l'appareil de radiologie. Le système de ponction comprend en général une aiguille pour effectuer le prélèvement dans une zone suspectée d'être cancéreuse, aux fins d'analyse et un porte-aiguille. L'appareil de radiologie sert alors à assurer le positionnement de l'aiguille. Le système de ponction peut également servir à la pose d'un hameçon équipé d'un fil destiné au repérage par le chirurgien d'une zone cancéreuse lors d'une opération.

Lors de l'utilisation du système de ponction, on effectue en général un premier cliché de centrage de la zone à ponctionner, puis, grâce à un mécanisme de basculement de la source de rayons X, on effectue un cliché avec un angle de +15° et un second cliché avec un angle de -15° dans le but d'obtenir par stéréotaxie les coordonnées tridimensionnelles d'un point particulièrement intéressant, puis on procède, après l'entrée de l'aiguille dans l'organe, à au moins deux clichés de contrôle pour vérifier que l'aiguille est en place dans la zone à ponctionner. Dans la pratique, on arrive à un total de l'ordre de huit clichés lors de l'utilisation du système de ponction. Le développement d'un film impressionnable dure de 3 à 5 minutes par cliché.

Pendant toute la durée de l'opération de biopsie et du développement des clichés, l'organe reste parfaitement immobile par rapport à l'appareil de radiologie et est maintenu en compression entre la table et le plateau de maintien. La patiente doit donc rester plus de 30 minutes dans une position immobile et relativement pénible.

Dans le but de réduire le temps d'immobilisation de l'organe, les cassettes contenant des films impressionnables peuvent être remplacées par des cassettes contenant un moyen de prise d'images numériques capable de réaliser une prise d'images extrêmement rapide. Les opérations de biopsie sont ainsi beaucoup plus brèves et réduisent l'inconfort de ces examens. De plus, les cassettes de prise d'images numériques permettent une amélioration de la qualité du diagnostic. Pour des raisons économiques, il est souhaitable d'utiliser des cassettes de prise d'images numériques sans changer le reste de l'appareil de radiologie. La cassette doit être amovible de façon à pouvoir être disposée soit sous la table lors d'un diagnostic ou dans le système de ponction lors d'une biopsie.

Une cassette de prise d'images numériques comprend une enveloppe à l'intérieur de laquelle est disposé un dispositif de détection du signal radiographique. Ce dispositif peut, par exemple, comprendre un scintillateur capable de transformer le rayonnement X incident en rayonnement lumineux, une fibre optique permettant de filtrer la majeure partie du rayonnement X ayant traversé le scintillateur et protégeant les composants situés après ladite fibre optique, et une caméra matricielle à éléments de transfert de charge (CCD) formant une zone sensible. Ce dispositif de détection est relativement onéreux et fragile. Or il est indispensable d'obtenir une image de l'organe radiographié jusqu'au plus près du thorax de la patiente. La zone sensible de la cassette doit donc être disposée sur un bord de celle-ci de façon à venir à proximité du thorax de la patiente.

L'inconvénient de ce type de cassette (voir EP 0 714 038) dont la zone sensible, fragile et onéreuse est située sur un bord, est le risque de détérioration, par exemple par percussion sur la zone sensible, lors d'une manipulation de la cassette par un opérateur.

La présente invention a pour objet la réalisation d'une cassette dont la zone sensible est protégée contre les chocs mécaniques lorsque la cassette n'est pas en service tout en permettant l'obtention d'une image de l'organe prise au plus près du thorax.

Le dispositif de cassette amovible de prise d'images numériques destiné à la radiographie par rayons X, selon l'invention, est défini dans la revendication 1.

Grâce à l'invention, on obtient une réduction sensible du coût dû à la détérioration de cassettes lors de manipulation par un opérateur, la partie la plus onéreuse et la plus sensible de la cassette étant bien protégée.

Dans un mode de réalisation de l'invention, le dispositif de prise d'images comprend une boîte sensiblement parallélépipédique de support des moyens capteurs des rayons X, lesdits moyens capteurs étant disposés à l'intérieur de la boîte et sur un des côtés de celle-ci.

Dans un mode de réalisation de l'invention, le dispositif de prise d'images comprend un étui muni d'une face ouverte et capable de recevoir la boîte, le côté de la boîte solidaire des moyens capteurs étant capable de venir en contact avec la face de l'étui opposé à ladite face ouverte. On peut ainsi réaliser une radiographie au plus près du thorax de la patiente.

Dans un mode de réalisation de l'invention, le dispositif de prise d'images comprend des moyens élastiques disposés entre le fond de l'étui formé par la face opposée à la face ouverte et le côté de la boîte solidaire des moyens capteurs pour écarter le boîte du fond de l'étui en position de repos, ledit côté de la boîte étant capable d'entrer en contact avec le fond de l'étui, les moyens élastiques n'étant pas en butée. Ainsi, lors d'un choc dû à une manipulation, la boîte peut se déplacer par rapport à l'étui en diminuant ainsi l'accélération due au choc subi par les moyens capteurs. La boîte peut comprendre au moins un logement des moyens élastiques formés par au moins un ressort.

De préférence, le côté de la boîte opposé au côté solidaire des moyens capteurs, fait saillie par rapport à la face ouverte de l'étui, le dispositif étant en position de repos.

Dans un mode de réalisation de l'invention, la boîte est capable de se déplacer en translation par rapport à l'étui.

Dans un autre mode de réalisation, la boîte est capable de se déplacer par rapport à l'étui par pivotement autour d'un coin de la boîte en contact avec le fond de l'étui.

Dans un mode de réalisation de l'invention, les moyens pour transformer les rayons X en signaux électriques comprennent un scintillateur capable de transformer les rayons X en rayonnement de lumière visible, une fibre optique, et une caméra matricielle CCD.

Le système de radiographie, selon l'invention, comprend un dispositif de prise d'images, un moyen d'émission de rayons X, un support du dispositif de prise d'images, l'organe à radiographier étant disposé entre le support et le moyen d'émission, et un moyen de commande et de traitement des signaux relié au dispositif de prise d'images pour exploiter les images obtenues. Dans un mode de réalisation de l'invention, le système comprend, en outre, un moyen pour effectuer une biopsie dans l'organe radiographié.

Grâce à l'invention, on obtient un système de radiologie capable de fournir des images de l'organe radiographié en un laps de temps très bref tout en permettant une amélioration de la qualité du diagnostic avec un coût d'utilisation réduit.

L'invention sera mieux comprise à l'étude de la description détaillée de quelques modes de réalisation pris à titre d'exemples nullement limitatifs et illustrés par les dessins annexés sur lesquels :
la figure 1 est une vue d'ensemble en perspective du système de mammographie;
la figure 2 est une vue de dessus en élévation du dispositif de prise d'images en position de repos;
la figure 3 est une vue schématique en coupe transversale de la zone sensible du dispositif de prise d'images;
la figure 4 est une vue en perspective d'un dispositif de prise d'images à proximité de son logement;
la figure 5a est une vue de dessus en élévation du dispositif de prise d'images en position de repos;
la figure 5b est une vue de dessus du dispositif de prise d'images inséré dans son logement; et
la figure 6 est une vue de dessus en élévation d'un autre mode de réalisation du dispositif de prise d'images.

Tel qu'il est illustré sur les figures, le système de mammographie comprend un mammographe 1, un système de ponction 2, une cassette 3 de prise d'images numériques et un moyen de commande et de traitement 4. L'appareil de radiologie comprend une base 5 reposant sur le sol et supportant un plateau porte-sein 6 de hauteur réglable et une source de rayons X 7 qui peut être basculée de ± 15° par rapport au plan vertical de symétrie de l'appareil de radiologie 1. La source de rayons X 7 est supporté par une colonne 8 pourvue sur sa face avant d'une pluralité de trous 9 de fixation du système de ponction 2.

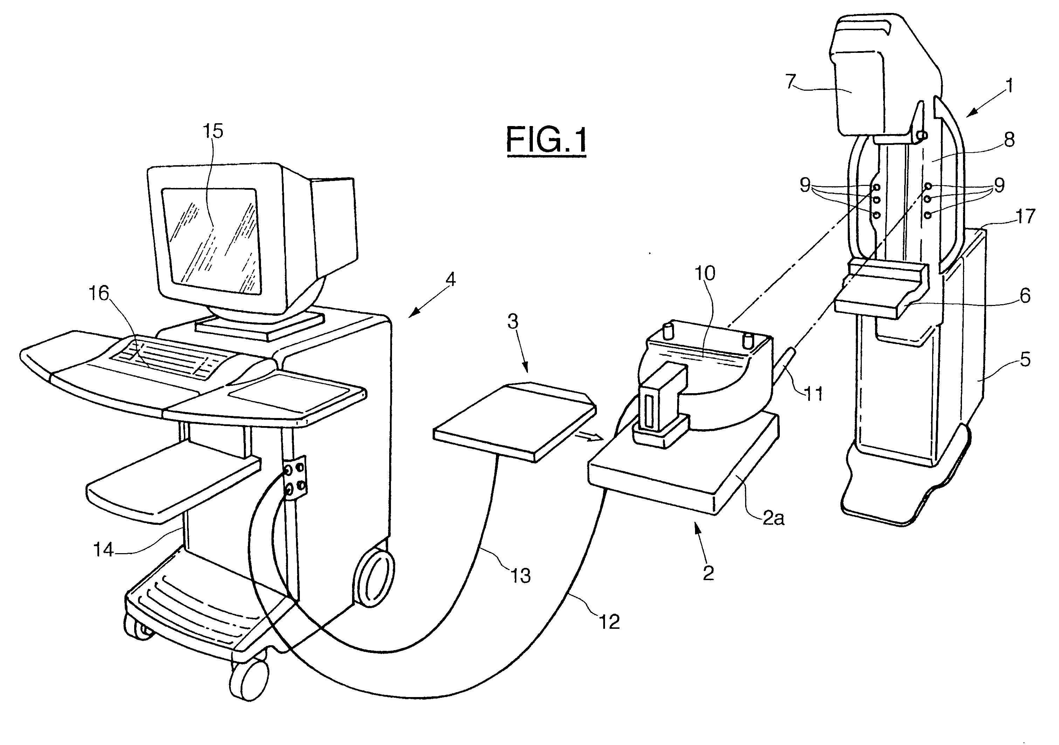

Le système de ponction 2 peut être monté ou non sur l'appareil de radiologie 1 et comprend un plateau de maintien 2a jouant le rôle de pelote de compression, un porte-aiguille 10 et une aiguille non représentée capable d'effectuer une biopsie dans l'organe à radiographier. Le système de ponction 2 est équipé de deux broches 11, dont une seule est visible sur la figure 1, capables de venir se loger dans les trous 9 de la colonne 8 du mammographe 1 et de grenouillères, non représentées, de fixation sur la colonne 8. Le système de ponction 2 est relié par un câble électrique 12 au moyen de commande et de traitement 4.

La cassette 3 de prise d'images numériques est plate et sensiblement parallélépipédique, et est reliée par un câble électrique 13 au moyen de commande et de traitement 4.

Le moyen de commande et de traitement 4 comprend un châssis 14 et des moyens électroniques non représentés reliés au système de ponction 2 et à la cassette 3 respectivement par les câbles électriques 12 et 13 pour traiter les informations reçues de la cassette 3 et commander le système de ponction 2, en particulier, le déplacement du porte-aiguille 10 lors d'une biopsie. Le moyen de commande et de traitement 4 comprend également un écran 15 de visualisation des images de l'organe radiographié et un clavier 16. Le moyen de commande et de traitement 4 peut être équipé d'un logiciel destiné au calcul des coordonnées tridimensionnelles de points de l'organe radiographié à partir de deux images prises selon des angles différents grâce au pivotement de la source 7 de rayons X. On peut alors obtenir une excellente visualisation soit de zones particulières de l'organe radiographié lors d'un diagnostic, soit du positionnement de l'aiguille dans l'organe radiographié lors d'une biopsie, en utilisant des méthodes d'affichage optimisées.

La cassette 3 peut entrer en suivant le sens de la flèche de la figure 1 dans un logement du système de ponction 2, ou dans un logement prévu dans un porte-cassette, non représenté, utilisé lors des examens de diagnostic et prévu pour être fixé sur le plateau porte-sein 6, ou encore en position de repos, dans un rangement 17 du mammographe 1. Le fait de prévoir un rangement 17 sur le mammographe 1 permet d'utiliser un câble 13 de faible longueur et de réduire ainsi les risques de chute et de détérioration de la cassette 3.

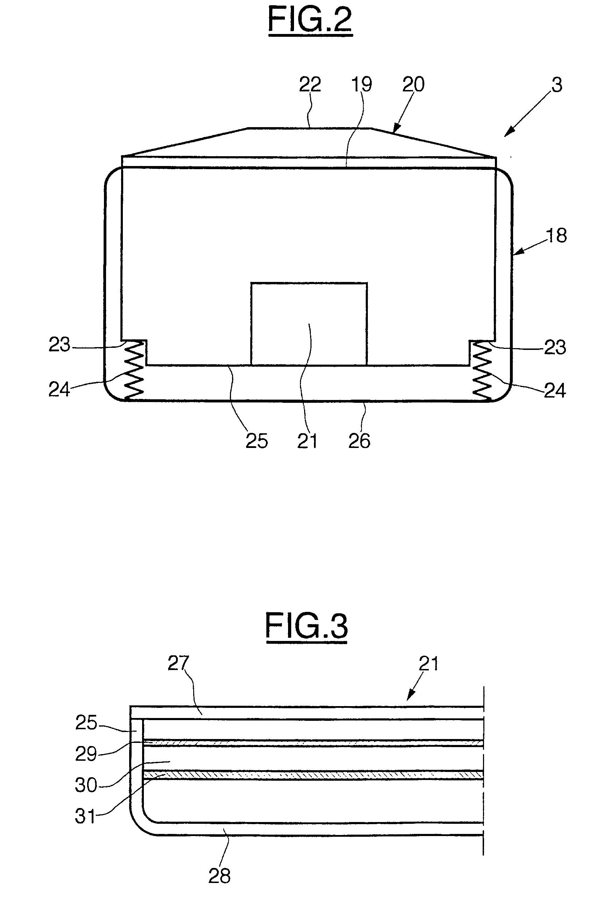

Telle qu'elle est illustrée sur la figure 2, la cassette 3 comprend un étui 18 de faible épaisseur, sensiblement parallélépipédique et pourvue d'une face ouverte 19. A l'intérieur de l'étui 18 est logée une boîte 20 de dimensions adaptées. La boîte 20 comprend une zone sensible 21, une portion en saillie 22 par rapport à la face ouverte 19 de l'étui 18 et deux logements 23 de ressorts 24. Les logements 23 sont prévus sur la face 25 de la boîte 20 opposée à la portion en saillie 22. Les deux ressorts hélicoïdaux 24 sont disposés entre le fond 26 de l'étui 18 et les logements 23 et tendent à écarter le fond 26 de la face 25 de la boîte 20 qui lui fait face. La face ouverte 19 peut éventuellement être recouverte d'une membrane souple destinée à prévenir l'intrusion de corps étrangers dans la cassette 3. Une telle membrane ne modifierait pas le fonctionnement de ladite cassette 3.

La zone sensible 21 de la boîte 20 est disposée à proximité de la face 25 de façon à être protégée par le fond 26 de l'étui 18. Ainsi, la zone sensible 21, protégée par le fond 26, ne peut pas entrer directement en contact avec un objet extérieur lors d'un choc dû à une manipulation.

Telle qu'illustrée sur la figure 3, la zone sensible 21 comprend une paroi supérieure 27 transparente aux rayons X, une partie de la face 25 et une paroi inférieure 28. Entre la paroi supérieure 27 et la paroi inférieure 28 sont disposés un scintillateur 29 capable de transformer les rayons X en lumière visible, une couche de fibre optique 30 destinée au transfert de la lumière visible et une caméra matricielle 31 composée d'une pluralité de cellules à transfert de charge appelées CCD.

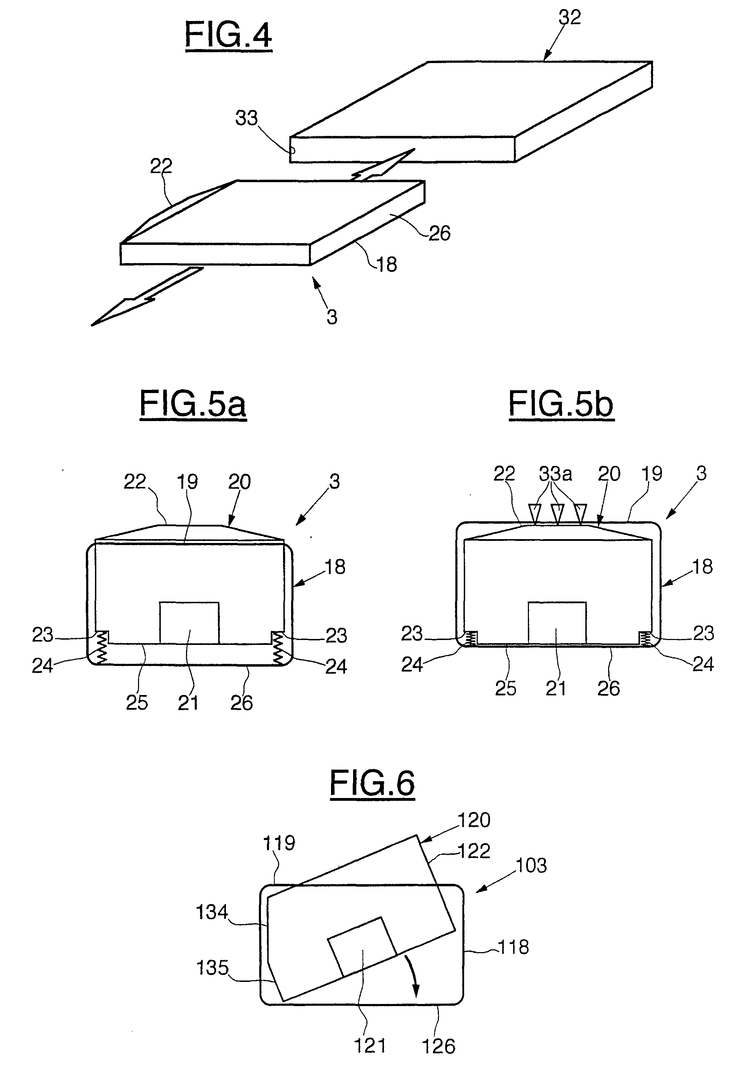

En fonctionnement, les rayons X sont émis par la source 7 (figure 1), traversent le plateau de maintien 2a du système de ponction 2, l'organe radiographié, le porte-cassette, la paroi supérieure 27 de la zone sensible 21 de la cassette 3 et passent dans le scintillateur 29 lequel, à la réception de rayons X, émet de la lumière visible transférée à la caméra matricielle 31 par la couche de fibre optique 30. La caméra matricielle 31 permet la transformation de l'information reçue sous forme de lumière visible en une information sous la forme d'un signal électrique transmis par le câble électrique 13 au moyen de commande et de traitement 4 (figure 4). La zone sensible 21 étant, en fonctionnement, située sur le bord de la cassette 3, on obtient une image qui peut comprendre les zones du sein les plus proches du thorax de la patiente.

Le système de ponction 2 illustré sur la figure 1 et le porte-cassette utilisé lors des examens de diagnostic comprennent chacun un logement 32 (figure 4) capable de recevoir une cassette 3. La cassette 3 peut entrer ou sortir du logement 32 dans le sens indiqué par les flèches. Le logement 32 est équipé de moyens de guidage, non représentés, appropriés à la cassette 3. La cassette 3 est présentée à l'entrée du logement 32 de façon qu'un des petits côtés 33 du logement 32 vienne en contact avec la partie en saillie 22 à l'opposé du fond 26 de l'étui 18. Lors de l'insertion de la cassette 3 dans le logement 32, le petit côté 33 vient porter contre la partie en saillie 22 et provoque l'effacement de la partie en saillie 22 dans l'étui 18 en comprimant les ressorts 24 sans que ceux-ci ne viennent en butée.

Ce mouvement de translation est illustré de façon schématique sur les figures 5a et 5b. On voit qu'avant l'entrée de la cassette 3 dans le logement 32, la zone sensible 21 est éloignée du fond 26 de l'étui 18. Au contraire après insertion de la cassette 3 dans le logement 32 (figure 5b), une portion d'appui 33a du côté 33 du logement 32 vient porter sur la portion en saillie 22 et provoque son effacement dans l'étui 18. Le côté 25 de la boîte 20 vient en contact avec le fond 26 de l'étui 18 et la zone sensible 21 se trouve ainsi à proximité immédiate du fond 26 de l'étui 18 ce qui permet de réaliser la prise d'images numériques de façon satisfaisante.

Sur la figure 6, les références des éléments semblables à ceux des figures précédentes ont été augmentées du nombre 100. L'étui 118 de la cassette 103 est inchangé. La boîte 120 comprend un coin tronqué 134 et un coin d'appui 135. La boîte 120 est en contact permanent avec le fond 126 de l'étui 118 par le coin d'appui 135. En position de repos, le coin opposé au coin d'appui 135 forme la portion en saillie 122 sous l'effet de moyens élastiques non représentés. Le coin tronqué 134 est lui à proximité de la paroi de l'étui 118. Lors de l'insertion dans un logement prévu à cet effet, la portion en saillie 122 est rabattue à l'intérieur du l'étui 118. La boîte 120 pivote dans le sens indiqué par la flèche et la zone sensible 121 vient se placer à proximité du fond 126 de l'étui 118.

On réalise ainsi, grâce à l'invention, une cassette de prise d'images numériques adaptable à des appareils de radiologie existants, et particulièrement bien protégée contre les détériorations dues aux manipulations par les opérateurs grâce à l'étui et à la boîte mobiles l'un par rapport à l'autre et à la protection de la zone sensible par le fond de l'étui. Si l'étui est seul détérioré, on pourra le remplacer tout en conservant la même boîte. Or l'étui est d'un coût faible par rapport à la boîte et à la zone sensible. On réduit ainsi les coûts de fonctionnement du système de radiographie.

## Revendications

1. Dispositif *de cassette* amovible (3) de prise d'images numériques destiné à la radiographie par rayons X, comprenant des moyens capteurs des rayons X, disposés *à l'intérieur et* sur un bord *de la cassette* lors de la prise d'images, **caractérisé par le fait qu'**il comprend un moyen pour *déplacer* les moyens capteurs des rayons X lorsque la cassette n'est pas en service en écartant lesdits moyens capteurs des rayons X du bord *de la cassette pour protéger les moyens capteurs lors de manipulations de la cassette.*

2. Dispositif de cassette selon la revendication 1, **caractérisé par le fait qu'**il comprend une boîte (20) sensiblement parallélépipédique de support des moyens capteurs des rayons X, lesdits moyens étant disposés à l'intérieur de la boîte et sur un des côtés de celle-ci.

3. Dispositif de cassette selon la revendication 2, **caractérisé par le fait qu'**il comprend un étui (18) muni d'une face ouverte (19) et capable de recevoir la boîte (20), le côté (25) de la boîte solidaire des moyens capteurs des rayons X étant capable de venir en contact avec la face de l'étui (18) opposée à ladite face ouverte (19).

4. Dispositif de cassette selon la revendication 3, **caractérisé par le fait qu'**il comprend des moyens élastiques disposés entre le fond (26) de l'étui (18) formé par la face opposée à la face ouverte (19) et le côté (25) de la boîte (20) solidaire des moyens capteurs des rayons X, pour écarter la boîte du fond de l'étui en position de repos, ledit côté de la boîte étant capable d'entrer en contact avec le fond de l'étui, les moyens élastiques n'étant pas en butée.

5. Dispositif de cassette selon la revendication 4, **caractérisé par le fait que** la boîte (20) comprend au moins un logement (23) des moyens élastiques formés par au moins un ressort (24).

6. Dispositif de cassette selon la revendication 3, 4 ou 5, **caractérisé par le fait que** le côté de la boîte (20) opposé au côté solidaire des moyens capteurs des rayons X, fait saillie par rapport à la face ouverte (19) de l'étui (18), le dispositif étant en position de repos.

7. Dispositif de cassette selon l'une des revendications 3 à 6, **caractérisé en ce que** la boîte (20) est capable de se déplacer en translation par rapport à l'étui (18).

8. Dispositif de cassette selon l'une des revendications 3 à 6, **caractérisé par le fait que** la boîte (120) est capable de se déplacer par rapport à l'étui par pivotement autour d'un coin (135) de la boîte en contact avec le fond de l'étui (118).

9. Dispositif de cassette selon l'une quelconque des revendications précédentes, **caractérisé par le fait que** les moyens capteurs des rayons X comprennent un scintillateur (29) capable de transformer les rayons X en rayonnement de lumière visible, une fibre optique (30), et une caméra matricielle CCD (31).

10. Système de radiographie comprenant un dispositif de cassette amovible de prise d'images selon l'une quelconque des revendications précédentes, **caractérisé par le fait qu'**il comprend un moyen d'émission (7) de rayons X, un support (6) du dispositif de prise d'images, l'organe à radiographier étant disposé entre ledit support et ledit moyen d'émission, et un moyen de commande et de traitement (4) des signaux relié au dispositif de prise d'images pour exploiter les images obtenues.

11. Système de radiographie selon la revendication 10, **caractérisé par le fait qu'**il comprend, en outre, un moyen (2) pour effectuer une biopsie dans l'organe radiographié.

## Patentansprüche

1. Abnehmbare Kassettenvorrichtung (3) zur Aufnahme von digitalen Bildern, bestimmt für die Röntgenstrahlen-Radiographie, umfassend Mittel zur Aufnahme von Röntgenstrahlen, welche Mittel während der Bildaufnahme im Innern und auf einem Rand der Kassette angebracht sind; **dadurch gekennzeichnet dass** sie ein Mittel zum Verschieben der Mittel zur Aufnahme von Röntgenstrahlen umfasst, wenn die Kassette nicht in Betrieb ist, wobei die Mittel zur Aufnahme von Röntgenstrahlen von dem Rand der Kassette getrennt werden, um die Mittel zur Aufnahme von Röntgenstrahlen beim Hantieren mit der Kassette zu schützen.

2. Kassettenvorrichtung nach Anspruch 1, **dadurch gekennzeichnet dass** sie einen näherungsweise parallelepipedischen Kasten (20) zum Abstützen der Mittel zur Aufnahme von Röntgenstrahlen umfasst, wobei die besagten Mittel im Innern des Kastens und auf seinem Rand angebracht sind.

3. Kassettenvorrichtung nach Anspruch 2, **dadurch gekennzeichnet dass** sie eine Hülle (18) umfasst, die eine offene und zum Aufnehmen des Kastens (20) geeignete Fläche (19) aufweist, wobei die Seite (25) des mit den Mitteln zur Aufnahme von Röntgenstrahlen verbundenen Kastens geeignet ist, mit der der besagten offenen Fläche (19) gegenüberliegenden Fläche der Hülle (18) in Kontakt zu kommen.

4. Kassettenvorrichtung nach Anspruch 3, **dadurch gekennzeichnet dass** sie elastische Mittel umfasst, die zwischen der durch die der offenen Seite (19) gegenüberliegenden Seite gebildeten Rückseite (26) der Hülle (18) und der Seite (25) des Kastens (20), die mit den Mitteln zur Aufnahme von Röntgenstrahlen verbunden ist, angebracht sind, um in einer Ruhestellung den Kasten von der Rückseite der Hülle zu trennen, wobei die besagte Seite des Kastens geeignet ist, mit der Rückseite der Hülle in Kontakt zu treten, wenn die elastischen Mittel ihren Anschlag nicht erreicht haben.

5. Kassettenvorrichtung nach Anspruch 4, **dadurch gekennzeichnet dass** der Kasten (20) mindestens eine Lagerung (23) für die durch mindestens eine Feder (24) gebildeten elastischen Mittel umfasst.

6. Kassettenvorrichtung nach einem der Ansprüche 3, 4 oder 5, **dadurch gekennzeichnet dass** die Seite des Kastens (20), die der mit den Mitteln zur Aufnahme von Röntgenstrahlen verbundenen Seite gegenüber liegt, in Bezug auf die offene Seite (19) der Hülle (18) hinausragt, wenn die Vorrichtung in einer Ruhestellung ist.

7. Kassettenvorrichtung nach einem der Ansprüche 3 bis 6, **dadurch gekennzeichnet dass** der Kasten (20) geeignet ist, sich in Bezug auf die Hülle (18) in Translation zu verschieben.

8. Kassettenvorrichtung nach einem der Ansprüche 3 bis 6, **dadurch gekennzeichnet dass** der Kasten (120) geeignet ist, sich in Bezug auf die Hülle durch Drehung um eine Ecke (135) des Kastens in Kontakt mit der Rückseite der Hülle (118) zu bewegen.

9. Kassettenvorrichtung nach einem der vorhergehenden Ansprüche, **dadurch gekennzeichnet dass** die Mittel zur Aufnahme von Röntgenstrahlen einen zur Umwandlung von Röntgenstrahlung in sichtbare Licht-Strahlung geeigneten Szintillator (29), eine optische Faser (30) und eine CCD-Matrix-Kamera (31) umfassen.

10. Radiografisches Aufnahmesystem, das eine abnehmbare Kassettenvorrichtung zur Bildaufnahme nach einem der vorergehenden Ansprüche umfasst, **dadurch gekennzeichnet dass** es ein Mittel (7) zur Emission von Röntgenstrahlen; eine Stütze (6) für das Bildaufnahmesystem, wobei das zu radiographierende Organ zwischen besagter Stütze und besagten Mitteln zum Ausstrahlen von Röntgenstrahlen angebracht ist; und zur Nutzung der erhaltenen Bilder mit dem Bildaufnahmesystem verbundene Mittel zur Kontrolle und Signal-Verarbeitung (4) umfasst.

11. Radiografisches Aufnahmesystem nach Anspruch 10, **dadurch gekennzeichnet dass** es ausserdem ein Mittel (2) zur Durchführung einer Biopsie in einem radiografierten Organ umfasst.

## Claims

1. Removable cassette device (3) for producing digital images, intended for X-ray radiography, comprising X-ray detector means arranged in the interieur and on an edge of the cassette during the producing of images, **characterized in that** it comprises a means for displacing the X-ray detector means by separating the said X-ray detector means from the edge of the cassette when the cassette is not in service, for protecting the X-ray detector means during handling of the cassette.

2. Cassette device according to claim 1, **characterized in that** it comprises an essentially parallelepipedal box (20) for supporting the X-ray detector means, the said means being arranged inside the box and on one of its edges.

3. Cassette device according to claim 2, **characterized in that** it comprises a case (18) provided with an open face (19) and capable of receiving the box (20), the side (25) of the case integral with the X-ray detector means being capable of coming into contact with the face of the case (18) opposite the said open face (19).

4. Cassette device according to claim 3, **characterized in that** it comprises elastic means arranged between the back (26) of the case (18) formed by the face opposite the open face (19) and the side (25) of the box (20) integral with the X-ray detector means, for separating the box from the back of the case in an inactive position, said side of the box being capable of entering in contact with the back of the case, when the elastic means have not reached the end of their range of movement.

5. Cassette device according to claim 4, **characterized in that** the box (20) comprises at least one housing (23) for the elastic means formed by at least one spring (24).

6. Cassette device according to claim 3, 4 or 5, **characterized in that** the side of the box (20) opposite the side integral with the X-ray detector means, projects relative to the open face (19) of the case (18) when the device is in an inactive position.

7. Cassette device according to anyone of the claim 3 to 6, **characterized in that** the box (20) is capable to be moved in translation relative to the case (18).

8. Cassette device according to anyone of the claim 3 to 6, **characterized in that** the box (120) is capable to be pivotally moved relative to the case (118) about a corner (135) of the box (120) in contact with the back of the case (118).

9. Cassette device according to anyone of the preceding claims, **characterized in that** the X-ray detector means comprise a scintillator (29) capable to transform X-ray in visible light radiation, an optical fibre (30) and a CCD matrix camera (31).

10. Radiography system comprising a removable cassette device for producing images according to anyone of the preceding claims, **characterized in that** it comprises a means for emitting X-rays (7), a support (6) for the device for producing images, the organ to be radiographed being arranged between the said support and the emission means, and a control and signal-processing means (4) connected to the device for producing images to utilize the images obtained.

11. Radiography system according to claim 10, **characterized in that** it furthermore comprises a means (2) for performing a biopsy in the radiographed organ.
